# EUROPEAN PATENT APPLICATION

(11) **EP 4 365 586 A1**
(43) Date of publication of application: **08.05.2024**
(21) Application number: 22828045.9
(22) Date of filing: 08.04.2022
(51) Int. Cl.: G01N 27/62

(54) **METHOD FOR ANALYZING ANTIBODY**

(30) Priority: 23.06.2021 US 202163213852 P
(71) Applicant: Shimadzu Corporation, Nakagyo-ku, Kyoto-shi, Kyoto 604-8511 (JP)
(72) Inventor: SHIMADA, Takashi, Columbia, Maryland 21046 (US); IWAMOTO, Noriko, Columbia, Maryland 21046 (US)
(74) Representative: Müller-Boré & Partner Patentanwälte PartG mbB
(86) International application number: PCT/JP2022/017389
(87) International publication number: WO 2022/270126

(57) **Abstract**

A method is provided that allows for antibody quantification with mass spectrometry without creating a calibration curve using an antibody of interest. An antibody analysis method is provided, including: digesting a biological protein and a target antibody with a protease; performing mass spectrometry of peptide fragments of the biological protein and the target antibody obtained by the digesting; and quantifying the target antibody based on a ratio between a detected intensity of the biological protein and a detected intensity of the target antibody obtained by the mass spectrometry, the digesting with a protease including: immobilizing the biological protein and the target antibody inside pores of a support; and bringing a microparticle close to the support, the microparticle having a diameter larger than a diameter of the pores and having a surface to which a protease is immobilized, wherein the biological protein has a domain capable of being immobilized inside the pores.

## Description

### TECHNICAL FIELD

The present invention relates to an antibody analysis method. More specifically, it relates to a method of antibody quantification utilizing mass spectrometry.

### BACKGROUND ART

To quantify a protein, ELISA (Enzyme-Linked ImmunoSorbent Assay) is used in general. However, ELISA has some disadvantages; it requires preparation of a specific antibody for each protein to detect, and it cannot detect a plurality of proteins simultaneously. To address these problems, researches have been conducted to develop a method of protein quantification with mass spectrometry, for replacing ELISA.

Our research group, to which the inventors of the present invention belong, found a method which involves regioselective protease digestion of a protein, such as an antibody, to obtain a peptide fragment that is optimum for specific detection of the protein with mass spectrometry. The peptide fragment thus obtained can be effectively detected and quantified by liquid chromatography-mass spectrometry (PTL 1: WO 2015/033479, PTL 2: WO 2016/194114, NPL 1: Iwamoto N et. al., Analyst. 2014 Feb 7; 139(3): 576-80. doi: 10.1039/c3an02104a).

### CITATION LIST

### PATENT LITERATURE

PTL 1: WO 2015/033479
PTL 2: WO 2016/194114

### NON PATENT LITERATURE

NPL 1: Iwamoto N et. al., Selective detection of complementarity-determining regions of monoclonal antibody by limiting protease access to the substrate: nano-surface and molecular-orientation limited proteolysis, Analyst. 2014 Feb 7; 139(3): 576-80. DOI: 10.1039/c3an02104a

### SUMMARY OF INVENTION

### TECHNICAL PROBLEM

To quantify an antibody with mass spectrometry, it is typical to create a calibration curve by using the antibody of interest. However, when a plurality of antibodies are to be measured, it is inefficient to create a calibration curve for each antibody. In addition, for an antibody such as a neutralizing antibody or a new clone that is important in functional analysis, it is often impossible to steadily obtain enough antibody for creating a calibration curve. The present invention provides a method that enables antibody quantification with mass spectrometry without creating a calibration curve using an antibody of interest.

### SOLUTION TO PROBLEM

The present invention relates to an antibody analysis method, comprising:
digesting a biological protein and a target antibody with a protease;
performing mass spectrometry of peptide fragments of the biological protein and the target antibody obtained by the digesting; and
quantifying the target antibody based on a ratio between a detected intensity of the biological protein and a detected intensity of the target antibody obtained by the mass spectrometry,
the digesting with a protease including:
   immobilizing the biological protein and the target antibody inside pores of a support; and
   bringing a microparticle close to the support, the microparticle having a diameter larger than a diameter of the pores and having a surface to which the protease is immobilized,
wherein the biological protein has a domain capable of being immobilized inside the pores.

The present invention relates to an antibody analysis method, comprising:
digesting a biological protein and a sample containing at least two target antibodies, with a protease,
performing mass spectrometry of peptide fragments of the biological protein and the target antibodies obtained by the digesting; and
quantifying each of the target antibodies based on a ratio between a detected intensity of the biological protein and a detected intensity of the each of the target antibodies,
wherein the biological protein is a monoclonal antibody.

The present invention relates to a program for use in the antibody analysis method, the program causing a processor to execute processes of:
receiving a mass spectrum output from a mass spectrometer;
retrieving a ratio between a detected intensity of the biological protein and a detected intensity of the target antibody, from a storage member;
correcting a detected amount of the biological protein calculated from the mass spectrum; and
calculating an amount of the target antibody.

### ADVANTAGEOUS EFFECTS OF INVENTION

The present invention enables antibody quantification using mass spectrometry, without necessity of creating a calibration curve using an antibody of interest.

### BRIEF DESCRIPTION OF DRAWINGS

Fig. 1 illustrates an example of a method of regioselective antibody cleavage.
Fig. 2 is a table listing signature peptide sequences and CDR3 sequences of antibodies used in Examples.
Fig. 3 is a table listing signature peptide sequences of antibodies used in Examples.
Fig. 4 describes the procedure of protease digestion of an antibody in Experiment 3.
Fig. 5 is a graph comparing an ion count (cps) obtained from mass spectrometry of a single sample, with cps of each antibody obtained from mass spectrometry of a multiplex sample, in Experiment 3. The horizontal axis indicates abbreviations for respective antibodies, and the vertical axis indicates cps.
Fig. 6 is a graph comparing a detected amount for a single sample after correction based on an internal reference, with a detected amount of each antibody in a multiplex sample after correction based on an internal reference, in Experiment 3. The horizontal axis indicates abbreviations for respective antibodies, and the vertical axis indicates values after correction based on the internal reference (internal reference corrected values).
Fig. 7 is a graph showing the ratio of the cps of each antibody to the cps of trastuzumab in Experiment 4. The horizontal axis indicates the concentration of each antibody, and the vertical axis indicates the ratio of the cps of each antibody to the cps of trastuzumab.
Fig. 8 is a graph showing the ratio of the cps of each antibody to the cps of trastuzumab in Experiment 4. The horizontal axis indicates the concentration of each antibody, and the vertical axis indicates the ratio of the cps of each antibody to the cps of trastuzumab.
Fig. 9 is a graph showing the ratio of the cps of each antibody to the cps of trastuzumab in Experiment 4. The horizontal axis indicates the concentration of each antibody, and the vertical axis indicates the ratio of the cps of each antibody to the cps of trastuzumab.
Fig. 10 is a graph comparing the ipilimumab concentration quantified based on a trastuzumab calibration curve (horizontal axis), with the ipilimumab concentration quantified based on an ipilimumab calibration curve (vertical axis), in Experiment 5.
Fig. 11 is a graph comparing the pembrolizumab concentration quantified based on a trastuzumab calibration curve (horizontal axis), with the pembrolizumab concentration quantified based on a pembrolizumab calibration curve (vertical axis), in Experiment 5.
Fig. 12 is a graph comparing the ipilimumab concentration quantified based on an ipilimumab calibration curve (horizontal axis), with the ipilimumab concentration quantified based on a trastuzumab calibration curve (vertical axis), in Experiment 6.
Fig. 13 is a graph comparing the pembrolizumab concentration quantified based on a pembrolizumab calibration curve (horizontal axis), with the pembrolizumab concentration quantified based on a trastuzumab calibration curve (vertical axis), in Experiment 6.
Fig. 14A is a graph comparing the ipilimumab concentration quantified based on an ipilimumab calibration curve (horizontal axes) with the ipilimumab concentration quantified based on a reference protein calibration curve (vertical axes), in Experiment 6.
Fig. 14B is a graph comparing the ipilimumab concentration quantified based on an ipilimumab calibration curve (horizontal axes) with the ipilimumab concentration quantified based on a reference protein calibration curve (vertical axes), in Experiment 6.
Fig. 15A is a graph comparing the pembrolizumab concentration quantified based on a pembrolizumab calibration curve (horizontal axes) with the pembrolizumab concentration quantified based on a reference protein calibration curve (vertical axes), in Experiment 6.
Fig. 15B is a graph comparing the pembrolizumab concentration quantified based on a pembrolizumab calibration curve (horizontal axes) with the pembrolizumab concentration quantified based on a reference protein calibration curve (vertical axes), in Experiment 6.

### DESCRIPTION OF EMBODIMENTS

### [Antibody Analysis Method]

An antibody analysis method according to the present embodiment includes:
digesting a biological protein and a target antibody with a protease;
performing mass spectrometry of peptide fragments of the biological protein and the target antibody obtained by the digesting; and
quantifying the target antibody based on a ratio between a detected intensity of the biological protein and a detected intensity of the target antibody obtained by the mass spectrometry.

The "biological protein" in the present embodiment means a protein derived from the living body such as cells or biological tissues or a protein obtained by synthesizing these artificially. In one aspect of the present embodiment, the above-mentioned biological protein is used as a reference substance in the antibody analysis method according to the present embodiment, and the biological protein can therefore also be identified as a "reference protein". The above-mentioned biological protein is preferably a biologic. The "biologic" in the present embodiment means a pharmaceutical produced, extracted, or synthesized using organisms. The above-mentioned biologic can also be identified as "biopharmaceutical" or "biological preparation". In another aspect of the present embodiment, examples of the above-mentioned biological protein include antibodies (for example, reference antibodies) and Fc fusion proteins described below and monoclonal antibodies derived from mice, rats, rabbits, or the like. In the present embodiment, the above-mentioned biological protein preferably includes a reference antibody or an Fc fusion protein.

In one aspect of the present embodiment, the above-mentioned biological protein preferably includes a reference antibody.

That is, an antibody analysis method according to the present embodiment preferably includes:
digesting a reference antibody and a target antibody with a protease;
performing mass spectrometry of peptide fragments of the reference antibody and the target antibody obtained by the digesting; and
quantifying the target antibody based on a ratio between a detected intensity of the reference antibody and a detected intensity of the target antibody.

The method according to the present embodiment, which does not require creating a calibration curve for each analyte, is expected to eliminate the use of antibody for calibration curve creation and simplify the procedure of mass spectrometry. In the practice of antibody pharmaceuticals, it is increasingly important to quantify an antibody in a living body (for example, in blood or tissue) in an easy and simple manner. By the present embodiment, even when it is impossible to create a calibration curve using an antibody of interest, it is still possible to calculate the concentration of an antibody pharmaceutical in a sample using other antibodies as a reference. In addition, the method according to the present embodiment can be implemented by using a single, generally-used antibody as a reference, and is, therefore, useful for simultaneously measuring two or more antibodies contained in a sample. It is expected that the method according to the present embodiment enables simplified pharmacokinetics tests as well as cost reduction.

In a living body, other than antibodies administered as medications (for example, monoclonal antibodies), there are also many antibodies produced in the living body. To create a calibration curve for an antibody produced in a living body is not easy, and therefore, conventionally, it has been difficult to quantify such an antibody by mass spectrometry. The method according to the present embodiment makes it possible to quantify neutralizing antibodies, autoantibodies, and their chronotypes produced in a living body, by using mass spectrometry.

### <Step of Digesting Antibody with Protease>

For easy, simple, and accurate detection and quantification of a protein with mass spectrometry, it is effective to regioselectively cleave the protein of interest to generate a peptide fragment unique to the protein in an efficient way, while limiting generation of other peptide fragments. When the protein of interest is an antibody, for example, regioselectively digesting the Fab domain or the variable region of the Fab domain while limiting digestion of the Fc domain can accomplish highly sensitive detection of the antibody.

In the method according to the present embodiment, the target antibody refers to an antibody to be quantified, and the reference antibody refers to an antibody used as a reference in quantification of the target antibody. The above-mentioned reference antibody corresponds to the above-mentioned biological protein. In the present specification, the target antibody and the reference antibody may be collectively called "antibody". An example of the antibody is immunoglobulin IgG, in which an Fc domain and a Fab domain are connected via a hinge portion. Each of the two heavy chains and the two light chains constituting the antibody molecule has a constant region and a variable region. The constant region has an amino acid sequence that is common to most antibodies derived from the same species. The variable region has three moieties called complementarity-determining regions (CDRs) which have specific sequences. The conformation determined by the CDRs (CDR1, CDR2, and CDR3) provides antigen specificity.

Protease digestion of the reference antibody and protease digestion of the target antibody may be sequentially performed, but preferably, they are performed simultaneously. The target antibody and the reference antibody may be contained in the same sample, or they may be contained in different samples.

The target antibody may include an antibody that has been manufactured, or may include an antibody produced in a living body. The target antibody may include a monoclonal antibody or a polyclonal antibody, for example. The target antibody may include an antibody produced in a living body by immune reaction, or may include a neutralizing antibody, an autoantibody, and/or a chronotype thereof. The target antibody may include two or more antibodies. The number of types of antibodies that can be simultaneously quantified may be 3, 4, 5, 6, 7, 8, 9, 10, 15, 20, 40, 60, 80, or more. The plurality of target antibodies may be contained in the same sample, or may be contained in different samples.

The target antibody may be an antibody contained in a cultured cell, a cultured tissue, or a culture supernatant thereof, and is preferably an antibody contained in a biological sample. The biological sample may be blood obtained from a subject, or may be biological tissue, urine, stool, sweat, saliva, lymph fluid, amniotic fluid, breast milk, pleural fluid, peritoneal fluid, cerebrospinal fluid, tear, and/or the like. The biological sample derived from the blood may be whole blood, preferably plasma or serum. The sample may be diluted with physiological saline, buffer, and/or the like. The subject may be an animal such as, for example, a mammal including a human. The subject may be a patient who received a monoclonal antibody as an antibody pharmaceutical. The biological sample may be obtained in a manner appropriate for the type of the biological sample, and after the sampling, it may be stored at room temperature or at a low temperature. When the target antibody is an antibody contained in a biological sample, the concentration of the target antibody in the living body can be determined by the method according to the present embodiment.

As the reference antibody, an antibody described above as an example of the target antibody can be used. The reference antibody is a different antibody from the target antibody. In one aspect of the present embodiment, the reference antibody can also be identified as an antibody in which at least the amino acid sequence of the variable region is different from that of the target antibody. Antibody used as a reference antibody in one analysis may be used as a target antibody in another analysis. Preferably, the concentration of the reference antibody is known. By using multiple concentrations of the reference antibody, a calibration curve can be created. The reference antibody may have a stable-isotope-labeled amino acid, or may have no labeling.

The reference antibody is preferably an antibody that is readily available. From the viewpoint of easily determining the concentration and the amino acid sequence, the reference antibody is preferably a monoclonal antibody. The monoclonal antibody may be, but not limited to, a fully human antibody, a humanized antibody, or a chimeric antibody, and examples include trastuzumab, trastuzumab-DM1, bevacizumab, cetuximab, rituximab, nivolumab, brentuximab, pembrolizumab, ipilimumab, mogamulizumab, ramucirumab, atezolizumab, durvalumab, avelumab, infliximab, tocilizumab, adalimumab, golimumab, mepolizumab, ustekinumab, eculizumab, panitumumab, ofatumumab, omalizumab, gemtuzumab, palivizumab, ranibizumab, certolizumab, ocrelizumab, and basiliximab. The molecular diameter of the monoclonal antibody is about 14.5 nm. Complexes that retain monoclonal antibody specificity and have an additional function, such as, for example, antibody-drug complexes (for example, brentuximab-vedotin, gemtuzumab-ozogamicin, and trastuzumab-emtansine), are also included as the monoclonal antibody for the method according to the present embodiment. Bonding that is present in such a complex may be dissociated before analysis so as to allow the antibody portion alone to be analyzed, or the complex can be analyzed as it is.

In the present embodiment, the above-mentioned biological protein may be an Fc fusion protein. The "Fc fusion protein" used here means a recombinant protein in which a functional protein or a domain thereof is fused with the Fc domain of an immunoglobulin. Examples of the above-mentioned functional protein or domain thereof include an extracellular domain of human cytototxic T-lymphocyte antigen-4 (CTLA-4), an extracellular domain of human vascular endothelial growth factor (VEGF) receptor 1 and 2 proteins, and tumor necrosis factor (TNF) receptor. Example of the above-mentioned Fc fusion protein include abatacept (fusion protein of the extracellular domain of CTLA-4 and the Fc domain of the immunoglobulin), aflibercept (the fusion proteins of the extracellular domain of VEGF receptor 1 and 2 proteins and the Fc domain of an immunoglobulin), and etanercept (fusion protein of TNF receptor and the Fc domain of an immunoglobulin). In one aspect of the present embodiment, it is preferable that the above-mentioned Fc fusion protein be selected from the group consisting of abatacept, aflibercept and etanercept.

In one aspect of the present embodiment, the step of digesting with a protease preferably includes the steps of:
(a) immobilizing the biological protein and the target antibody inside pores of a support; and
(b) bringing a microparticle close to the support, the microparticle having a diameter larger than a diameter of the pores and having a surface to which a protease is immobilized.

In another aspect of the present embodiment, for regioselectively digesting a Fab domain (or functional protein domain in the Fc fusion protein), the step of digesting an antibody with a protease preferably includes the steps of:
(a) immobilizing the reference antibody (or Fc fusion protein) and the target antibody inside pores of a support; and
(b) bringing a microparticle close to the support, the microparticle having a diameter larger than a diameter of the pores and having a surface to which the protease is immobilized.

### (a) Step of Immobilizing Reference Antibody and Target Antibody inside Pores of Support

The support for immobilizing the antibody may be a porous body having many pores inside which the antibody can be bonded. The material of the support may be, but not particularly limited to, activated carbon, porous film, porous resin beads, metal particles, and the like.

The shape of the pores may be, but not particularly limited to, hemispherical or a shape that penetrates through the support. The size of the pores is preferably selected in consideration of the antibody molecular diameter and the like so that, when the antibody is immobilized thereto, the top portion of the antibody (more specifically, the moiety to be selectively digested) is positioned near the opening of the pore. The average pore diameter of the support is selected as appropriate within the range of, for example, about 10 nm to 500 nm without exceeding the average particle diameter of the microparticles. The average pore diameter of the support may be, for example, 20 nm or more, 30 nm or more, 40 nm or more, or 50 nm or more, and 200 nm or less, 150 nm or less, 120 nm or less, or 100 nm or less. The average pore diameter of the support can be determined by electron microscopy.

For immobilizing the antibody inside the pores of the support, it is preferable to use a support that has a linker immobilized inside the pores and capable of site-specific interaction with the antibody. Examples of the interaction between the antibody and the linker include chemical bonding, hydrogen bonding, ionic bonding, complex formation, hydrophobic interaction, the van der Waals interaction, electrostatic interaction, and stereoselective interaction. From the viewpoint of facilitating immobilization inside the pores, preferably the reference antibody, more preferably both the reference antibody and the target antibody have a domain capable of being immobilized inside the pore. The domain capable of being immobilized inside the pore is, for example, an Fc domain of the antibody. In one aspect of the present embodiment, the biological protein has a domain capable of being immobilized in the pores.

As the linker, Protein A, Protein G, and the like capable of site-specific binding to an Fc domain of the antibody are preferably used. Using a support having the linker immobilized inside the pore allows the Fc domain of the antibody to be immobilized inside the pore, positioning the Fab domain near the opening of the pore. Controlling the orientation of the antibody inside the pore enables regioselective digestion of the Fab domain with a protease.

Examples of the support that may be suitably used in the present embodiment include, but not particularly limited to, Protein G Ultralink Resin (manufactured by Pierce), TOYOPEARL TSKgel (manufactured by TOSOH), TOYOPEARL AF-rProtein A HC-650F resin (manufactured by TOSOH), Protein A Sepharose (GE Healthcare), and KanCapA (KANEKA).

The method for immobilizing the antibody inside the pores of the support is not particularly limited, and when the antibody is to be immobilized to a support having pores having Protein A or Protein G immobilized inside thereof, for example, a suspension of the support can be mixed with a solution containing the antibody so as to facilitate immobilization of the antibody inside the pores. The ratio of the amount of the support to the amount of the antibody can be selected as appropriate in accordance with the purpose. Subsequently, the support is washed with a proper buffer to remove any non-specifically-bound antibody and molecules contained in the sample. As the buffer, Phosphate Buffered Saline (PBS), PBS containing n-octyl thioglycoside (OTG), and/or the like is used, for example. Washing may be carried out automatically for many samples at one time by, for example, aspiration or centrifugation with the use of a plate equipped with built-in filter.

### (a') Step of Performing Reduction Reaction under Acidic Conditions

Following the above step (a) of immobilizing the reference antibody and the target antibody inside the pores of the support, step (a') of performing reduction reaction on the antibodies under acidic conditions may be carried out. In some instances, an antibody can acquire protease resistance via some mechanism, for example, when S-S bonding (disulfide bonding) forms a cysteine knot structure to generate a very rigid region. For example, it is known that about 20 to 30% of monoclonal antibodies have localized protease resistance. Such a rigid antibody can be subjected to acidic reduction treatment to be rendered an enhanced protease digestion efficiency. It is considered that, under acidic reduction conditions, bonding of protein A or protein G to an Fc domain does not dissociate, allowing the cysteine knot structure to be loosened while the antibody molecule remains held to the support.

The reduction reaction under acidic conditions can be accomplished, for example, by incubation in the presence of an organophosphorus-based reducing agent. Examples of the organophosphorus-based reducing agent include Tris(2-carboxyethyl)phosphine (TCEP) or its hydrochloride, and tributylphosphine. These reducing agents are available from Sigma-Aldrich, Nacalai Tesque, Inc., Funakoshi Co., Ltd., and the like. The TCEP concentration in the reaction liquid may be, for example, from 100 mM to 500 mM. For example, the concentration is 250 mM.

Preferably, the reduction reaction is carried out under highly acidic conditions at pH2.5 or less such as, for example, at pH1.5, pH2.0, PH2.5, or the like. The duration of the reduction reaction is, but not limited to, within the range of 10 minutes to 60 minutes to be sufficient, and may be, for example, 20 minutes, 30 minutes, 40 minutes, 45 minutes, or 50 minutes. The duration of the reduction reaction may exceed 60 minutes, but in that case, more time is required for measurement. The reaction temperature may be, but not limited to, from 15°C to 30°C, for example, which means that the reaction can be carried out at room temperature. Preferably, the reaction temperature is about 25°C.

A sample without acidic reduction treatment and a sample with acidic reduction treatment may be simultaneously subjected to mass spectrometry. More specifically, the support and the antibody solution may be mixed together; the resulting mixed solution may be divided into two; one of the two may be directly washed and the other subjected to acidic reduction treatment before washing; both the washed supports may be combined and subjected to the subsequent step of protease digestion; and, in this way, a peptide fragment derived from the antibody with TCEP acidic reduction treatment and a peptide fragment derived from the antibody without TCEP acidic reduction treatment can be subjected to mass spectrometry as a single sample.

### (b) Step of Bringing Microparticle Close to Support, where Microparticle Has Diameter Larger than Diameter of Pore and Has Surface to which Protease Is Immobilized

The type of the protease to be immobilized to the microparticle may be selected as appropriate depending on the type of the antibody to be quantified by mass spectrometry, and, for example, trypsin, chymotrypsin, lysyl endopeptidase, V8 protease, AspN protease (Asp-N), ArgC protease (Arg-C), papain, pepsin, dipeptidyl peptidase can be used alone or in combination. Preferably, trypsin is used as the protease. Examples of the protease that can be suitably used in the method according to the present embodiment include Trypsin Gold (manufactured by Promega) and Trypsin TPCK-treated (manufactured by Sigma).

The microparticles are used for immobilizing the protease to its surface so as to limit the access of the protease to the antibody immobilized inside the pores of the support. For this reason, so as to prevent the microparticles from entering deep into the pores of the support, the microparticles used have an average particle diameter larger than the average pore diameter of the support. The average particle diameter of the microparticles may be 1.2 times or more, 1.5 times or more, 1.8 times or more, or about 2 times the average pore diameter of the support. The average particle diameter of the microparticles may be 50 nm or more, 100 nm or more, 120 nm or more, 150 nm or more, or 170 nm or more, and may be 500 nm or less or 300 nm or less. The average particle diameter of the microparticles may be determined by electron microscopy.

The shape of the microparticles is not particularly limited, and from the viewpoint of uniform access of the protease to the support, it is preferably spherical. Preferably, the microparticles are highly dispersible and have a uniform average particle diameter.

The material of the microparticles is not particularly limited as long as they are capable of immobilizing the protease to its surface, and metal, resin, and the like are used as appropriate. Also, microparticles of resin-surface-coated metal, microparticles of metal-surface-coated resin, and the like can be used.

Preferably, the microparticles are magnetic nanoparticles that can be dispersed or suspended in an aqueous medium and then easily recovered from the dispersion or suspension by magnetic separation or magnetic precipitation separation. From the viewpoint of reducing the likelihood of aggregation, the surface is preferably coated with organic polymer. Specific examples of magnetic nanobeads coated with organic polymer include FG beads, SG beads, Adembeads, and nanomag. Commercially available products like, for example, FG beads manufactured by Tamagawa Seiki Co., Ltd. (polymer magnetic nanoparticles with a particle diameter of about 200 nm, made by coating ferrite particles with polyglycidyl methacrylate (poly GMA)) are also preferably used.

For reducing non-specific protein adsorption and accomplishing selective protease immobilization, the microparticles are preferably modified with a spacer that is capable of binding to the protease. When the protease is immobilized via a spacer, protease detachment from the microparticle surface is reduced, enhancing regioselectivity of protease digestion. Through adjustment of the molecular diameter of the spacer, it is also possible to enable selective access of the protease to a desired position on the antibody for better regioselectivity.

Preferably, the spacer is capable of binding to the protease without inactivating the protease. From the viewpoint of controlling the range of protease access, the molecular diameter of the spacer is preferably small. The molecular diameter of the spacer is preferably 5 nm or less, more preferably 3 nm or less, further preferably 2 nm or less. The molecular weight of the spacer is preferably 2000 or less, more preferably 1500 or less, further preferably 1000 or less.

Preferably, the spacer is not a protein, and is preferably a molecule having a terminal functional group such as an amino group, a carboxyl group, an ester group, an epoxy group, a tosyl group, a hydroxyl group, a thiol group, an aldehyde group, a maleimide group, a succinimide group, an azide group, biotin, avidin, and/or chelate. To immobilize trypsin, for example, a spacer having an activated ester group is preferred. As the part of the spacer other than the functional group, namely as a spacer arm portion, a hydrophilic molecule is used, such as polyethylene glycol, polypropylene glycol, polyacrylamide, polyethyleneimine, poly(ethylene oxide), polyethylene terephthalate, and a derivative thereof.

Examples of the microparticle surface-modified with the spacer include a microparticle modified with a spacer having an ester group activated with N-hydroxysuccinimide (an active ester group), which is commercially available under the trade name of "FG beads NHS" (manufactured by Tamagawa Seiki Co., Ltd.).

The method for immobilizing the protease to a surface of the microparticle is not particularly limited, and any appropriate method can be adopted depending on, for example, the properties of the protease and the microparticle (or the spacer modifying the microparticle surface). For example, a suspension of a spacer-modified microparticle and a solution containing a protease can be mixed together to obtain a microparticle having a surface to which a protease is immobilized. As a commercially available microparticle to which trypsin is immobilized as the protease, "FG beads Trypsin DART (registered trademark)" contained in an LC/MS/MS pretreatment kit "nSMOL Antibody BA Kit" (manufactured by Shimadzu) can also be preferably used.

Bringing an antibody-immobilized support close to or into contact with a protease-immobilized microparticle allows for selective protease digestion of the variable region of the antibody.

Protease digestion can be carried out, for example, in a buffer solution having a pH adjusted to near the optimum pH of the protease. Protease digestion may be carried out preferably at a pH from 7 to 9, more preferably at about pH8.0. Protease digestion may be carried out under the environment at about 37°C, but preferably, it is carried out under saturated vapor pressure at about 50°C. The reaction time may be from 30 minutes to 20 hours, for example; it may be 1 hour or more or 3 hours or more, for example, and may be 5 hours or less or 8 hours or less. For preventing evaporation of the reaction liquid, the reaction is preferably carried out under saturated vapor pressure.

In the protease digestion step, the reaction liquid may be stirred so as to facilitate contact between the support and the microparticle. The reaction liquid may be stirred throughout the entire reaction time, or may be for only a part of the reaction time such as, for example, only during the initial stages of the reaction.

The peptide fragments generated by the protease digestion are released into the reaction liquid. For performing mass spectrometry of the peptide fragments, it is necessary to remove the support and the microparticle. This can be accomplished by performing such a process as filtration, centrifugation, magnetic separation, and/or dialysis of the reaction liquid after protease digestion.

Separation of the support and the microparticle from the peptide fragments can be accomplished in an easy and simple manner by, for example, filtration through a polyvinylidene difluoride (PVDF) membrane filter (Low-binding hydrophilic PVDF, 0.2 µm in pore size, manufactured by Millipore), a polytetrafluoroethylene (PTFE) membrane filter (Low-binding hydrophilic PTFE, 0.2 µm in pore size, manufactured by Millipore), and/or the like. Centrifugation can further be performed for fast, easy, and simple filtration.

In the above step of protease digestion of the antibody, nano-surface and molecular-orientation limited proteolysis (nSMOL) (registered trademark) previously developed by our research group (to which the inventors of the present invention belong) can be employed, and an LC/MS/MS pretreatment kit "nSMOL Antibody BA Kit" (manufactured by Shimadzu) can be suitably used. This nSMOL is described in detail in, for example, WO 2015/033479 and Iwamoto N et. al., Analyst. 2014 Feb 7;139(3):576-80, doi:10.1039/c3an02104a. Improved techniques and the like of nSMOL are disclosed in, for example, WO 2016/143223, WO 2016/143224, No. WO 2016/143226, WO 2016/143227, WO 2019/130549, WO 2019/155576, Iwamoto N et. al., Bioanalysis, doi:10.4155/bio-2016-0018, and Iwamoto N et. al., Biological&Pharmaceutical Bulletin, 2016, doi:10.1248/bpb.b16-00230. The disclosure of these documents are incorporated herein by reference.

An example protocol of nSMOL is as follows:
<Step (a)>
   1. Dilute 5 to 10 µL of an antibody-containing biological sample with about 10 to 20 times the amount of PBS + 0.1% n-octyl thioglycoside (OTG).
   2. Add 25 µL of Immunoglobulin Collection Resin (TOYOPEARL AF-rProtein A HC-650F, 50% slurry) to the diluted biological sample to obtain a liquid mixture.
   3. Stir the obtained liquid mixture gently with a vortex for about 5 minutes.
   4. Collect the whole content of the liquid mixture on Ultra-free low-protein binding Durapore PFDF (0.22 µm).
   5. Perform centrifugation to remove the supernatant (10000 g, 1 minute).
   6. Add 300 µL of PBS + 0.1% OTG, followed by centrifugation to remove the supernatant (10000 g, 1 minute).
   7. Repeat Step 6.
   8. For surfactant (OTG) removal, add 300 µL of PBS, followed by centrifugation to remove the supernatant (10000 g, 1 minute).
   9. Repeat Step 8.
   10. Add 75 to 100 µL of Reaction solution or Enhanced Reaction Solution.
   Optionally add 10 fmol/µL of P14R synthetic peptide to this solution.
<Step (b)>
   11. Add 5 to 10 µL of FG beads (0.5 mg/ml FG beads suspension) having chemically-modified trypsin immobilized thereto.
   12. Allow reaction to proceed for 4 to 6 hours under saturated vapor pressure at 50°C with gentle stirring.
   13. Add 10 µL of 10% formic acid to the reaction liquid to stop the reaction.
   14. Perform centrifugal filtration (10000 g, 1 minute) to recover a solution.
   15. Leave a tube containing the solution to stand on a magnetic stand for about 1 to 2 minutes to remove excess resin from the solution.

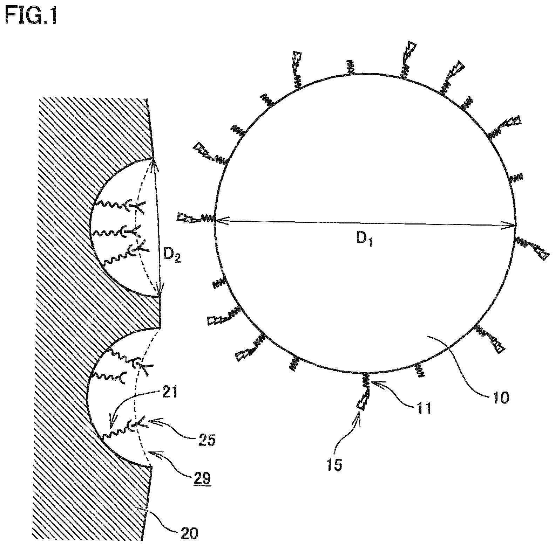

Fig. 1 illustrates an example of regioselective cleavage of an antibody by nSMOL. To a surface of a microparticle 10 with an average particle diameter D₁, a protease 15 is immobilized. Protease 15 is immobilized to the microparticle via a spacer 11. A support 20 has many pores 29 with an average pore diameter D₂, to which a linker 21 capable of specific antibody binding is immobilized. An antibody 25 is immobilized inside the pore via linker 21. The dotted line near pore 29 represents the boundary of the region accessible by protease 15. Because the access by the protease to an Fc domain of the antibody bonded to the interior surface of the pore is limited, the likelihood of access by the protease to a Fab domain located near the opening, farther from the binding position to the pore, is relatively increased. This enables regioselective protease cleavage of the Fab domain of the antibody to obtain peptide fragments.

### <Step of Mass Spectrometry of Peptide Fragments>

The peptide fragments obtained in the above step are subjected to mass spectrometry. Peptide fragments of the reference antibody and peptide fragments of the target antibody may be contained in the same sample, or may be contained in different samples. Peptide fragments derived from a plurality of target antibodies may be contained in the same sample, or may be contained in different samples.

For more reliable peptide fragment separation and more accurate analysis, it is preferable to perform liquid chromatography (LC) for separation and concentration of the sample prior to mass spectrometry. When LC is performed for sample separation, the LC eluate may be directly ionized for mass spectrometry. The analysis can also be carried out by LC/MS/MS or LC/MSn, each of which is a combination of LC with tandem mass spectrometry. The LC eluate may be subjected to fractionation before mass spectrometry. The LC column is not particularly limited, and can be selected as appropriate from, for example, hydrophobic columns (such as C30, C18, C8, and C4) and hydrophilic affinity chromatography supports commonly used in peptide analysis. When needed, before mass spectrometry, the sample may be subjected to treatment such as desalination, solubilization, extraction, concentration, and/or drying.

The method of ionization in mass spectrometry is not particularly limited, and electron ionization (EI), chemical ionization (CI), field desorption (FD), fast atom bombardment (FAB), matrix-assisted laser desorption ionization (MALDI), electrospray ionization (ESI), and/or the like may be employed. The method for analysis of ionized samples is not particularly limited, and can be selected as appropriate from magnetic sector type, quadrupole (Q) type, ion trap (IT) type, time-of-flight (TOF) type, Fourier transform ion cyclotron resonance (FT-ICR) type, and the like, depending on the selected ionization method. A triple quadrupole mass spectrometer and/or the like can be used to perform MS/MS analysis, multiple-stage mass spectrometry with MS3 or more, or multiple reaction monitoring (MRM).

Examples of the apparatus particularly suitable for the method according to the present embodiment can include, but not limited to, LCMS-8030, LCMS-8040, LCMS-8050, LCMS-8060, LCMS-9030, and LCMS-IT-TOF (all manufactured by Shimadzu).

Detecting peptide fragments including amino acid sequences of the Fab regions specific for the target antibody (for example, CDR1, CDR2, and CDR3 of the heavy chains and/or the light chains) with mass spectrometry allows for identifying the target antibody. For identifying the target antibody, an existing database can be used. Using the peptide fragments obtained by nSMOL (where the antibody is regiospecifically digested with protease) increases the database search hit rate and the data accuracy. Among a plurality of peptide fragments derived from the Fab region, peptide fragments that are particularly suitable for antibody identification can be selected as peptides for analysis. Those selected peptide fragments are called "signature peptides".

When antibody identification is to be performed based on the results of detection of particular peptide fragments that include CDR sequences, the peptide fragments to be detected are preferably of about 5 to 30 amino acid residues, more preferably about 7 to 25. When the number of amino acid residues is too small, they are not easily differentiated from a peptide fragment derived from foreign matter or from other moieties of the same protein, potentially leading to false detection and the like. When the number of amino acid residues is too high, ionization is difficult to perform, and this and some other issues may render the detection difficult or compromise the quantification properties.

### <Step of Target Antibody Quantification>

The target antibody is quantified with mass spectrometry based on a ratio between a detected intensity of the reference antibody and a detected intensity of the target antibody. The "detected intensity" in the present embodiment means the peak area or the peak intensity derived from the peptide fragment that is an object in a mass spectrum obtained by subjecting the peptide fragment to mass spectrometry. That is, the "detected intensity of the reference antibody" means the peak area or the peak intensity derived from the peptide fragment of the reference antibody in the mass spectrum obtained by subjecting the reference antibody to mass spectrometry. The "detected intensity of the target antibody" means the peak area or the peak intensity derived from the peptide fragment of the target antibody in the mass spectrum obtained by subjecting the target antibody to mass spectrometry. The target antibody can be quantified based on the ratio between the detected intensity of the reference antibody and the detected intensity of the target antibody, and based on the ratio between the detected amount of the reference antibody and the detected amount of the target antibody. The detected amount of the target antibody or the detected amount of the reference antibody can be corrected based on the ratio between the detected intensity of the reference antibody and the detected intensity of the target antibody. The detected amount of the reference antibody and the detected amount of the target antibody are calculated based on the peak area or the peak intensity of mass spectra obtained by mass spectrometry of peptide fragments of the reference antibody and the target antibody.

For example, based on the ratio between the detected intensity of the reference antibody and the detected intensity of the target antibody, the detected amount of the reference antibody can be converted to a standard amount for the target antibody, which can then be compared to the actual detected amount of the target antibody to accomplish quantification of the target antibody. For example, based on the ratio between the detected intensity of the reference antibody and the detected intensity of the target antibody, the detected amount of the target antibody can be converted to a corresponding, detected amount of the reference antibody, which can then be compared to the actual detected amount of the reference antibody (standard amount) to accomplish quantification of the target antibody. Mass spectrometry of the reference antibody may be performed at one concentration, or the mass spectrometry may be performed at a plurality of concentrations. When mass spectrometry of the reference antibody is performed at a plurality of concentrations, results of the mass spectrometry may be used to create a calibration curve for the reference antibody. For example, a calibration curve for the reference antibody may be converted to a calibration curve for the target antibody based on the ratio between the detected intensity of the reference antibody and the detected intensity of the target antibody, to accomplish quantification of the target antibody.

The ratio between the detected intensity of the reference antibody and the detected intensity of the target antibody can be determined by mass spectrometry of the reference antibody at a predetermined concentration and the target antibody at a predetermined concentration. For example, equal amounts of the reference antibody and the target antibody can be digested with protease under the same conditions, followed by mass spectrometry to obtain mass spectra, and the ratio in peak area or peak intensity can be used as the ratio between the detected intensity of the reference antibody and the detected intensity of the target antibody. As the ratio between the detected intensity of the reference antibody and the detected intensity of the target antibody, the median or average value of the ratios from five independent analyses may be used. Usually, the ratio between the detected intensity of the reference antibody and the detected intensity of the target antibody remains constant regardless of concentrations. The ratio between the detected intensity of the reference antibody and the detected intensity of the target antibody can be determined in advance. Determining it in advance allows for easy and simple target antibody quantification. The ratio between the detected intensity of the reference antibody and the detected intensity of the target antibody can be universally used as a constant for target antibody quantification.

That is, in one aspect of the present embodiment, preferably the above-mentioned antibody analysis method further includes:
performing mass spectrometry of the biological protein at a predetermined concentration and the target antibody at a predetermined concentration; and
determining in advance the ratio between a detected intensity of the biological protein and a detected intensity of the target antibody, based on results of the mass spectrometry.

In another aspect of the present embodiment, preferably the above-mentioned antibody analysis method further includes:
performing mass spectrometry of the reference antibody at a predetermined concentration and the target antibody at a predetermined concentration; and
determining in advance the ratio between a detected intensity of the reference antibody and a detected intensity of the target antibody.

The method of mass spectrometry of the reference antibody at a predetermined concentration and the target antibody at a predetermined concentration can be, but not particularly limited to, the method described above in the section for the step of mass spectrometry of the reference antibody and the target antibody. The mass spectrometry of the reference antibody at a predetermined concentration and the target antibody at a predetermined concentration may be performed simultaneously with the above step of mass spectrometry of the reference antibody and the target antibody, or may be performed before the above step of mass spectrometry of the reference antibody and the target antibody. Preferably, the step of mass spectrometry of the reference antibody and the target antibody is performed under the same conditions as those for the mass spectrometry of the reference antibody at a predetermined concentration and the target antibody at a predetermined concentration.

### [Kit]

A kit according to the present embodiment includes:
the biological protein;
the microparticle having a surface to which a protease is immobilized; and
the support provided with a pore.

This kit is a kit for use in the above-described antibody analysis method.

A kit according to the present embodiment includes:
a reference antibody;
a microparticle having a surface to which a protease is immobilized; and
a support provided with a pore.

This kit may be a kit for use in the above-described antibody analysis method.

The biological protein, the reference antibody, the microparticle, and the support may be those described for the above-described method. The kit may further include a reaction vessel such as a microplate and a tube, a plate equipped with built-in filter, a buffer, a wash liquid, a protease reaction liquid, an acidic reducing agent, a membrane filter, a chip, and an analysis column, which may be used in the above-described method. The kit may include instruction literature describing instructions for use of the kit and/or mass spectrometry conditions for antibody detection. The kit may also include a correspondence table showing the ratio between the detected intensity of the biological protein and the detected intensity of the target antibody, or a correspondence table showing the ratio between the detected intensity of the reference antibody and the detected intensity of the target antibody.

Using the kit according to the present embodiment allows for easier and simpler preparation of peptide fragments required for antibody detection, and may also allow for apparatus-based automation. Using the kit according to the present embodiment also allows for easy and simple mass spectrometry and target antibody quantification.

### [Program]

A program according to the present embodiment is a program for use in the antibody analysis method, wherein the program causes a processor to execute processes of:
receiving a mass spectrum output from a mass spectrometer;
retrieving a ratio between a detected intensity of the biological protein and a detected intensity of the target antibody, from a storage member;
correcting a detected amount of the biological protein calculated from the mass spectrum; and
calculating an amount of the target antibody.

A program according to the present embodiment causes a processor to execute processes of:
receiving a mass spectrum output from a mass spectrometer;
retrieving a ratio between a detected intensity of a reference antibody and a detected intensity of a target antibody, from a storage member;
correcting a detected amount of the reference antibody calculated from the mass spectrum; and
calculating an amount of the target antibody.

The program according to the present embodiment may be a program that executes the step of target antibody quantification in the above antibody analysis method.

An example of the program identifies the reference antibody and the target antibody in mass spectra output from the mass spectrometer, and calculates the detected amounts of the antibodies. The type and concentration of the reference antibody can be received from the user. In accordance with the reference antibody thus received, the ratio between the detected intensity of the reference antibody and the detected intensity of the target antibody is retrieved from a storage member. The ratio between the detected intensity of the reference antibody and the detected intensity of the target antibody may be a value determined by mass spectrometry of the reference antibody at a predetermined concentration and the target antibody at a predetermined concentration performed in the above-described manner. Preferably, this value is stored in the storage member in advance. Based on the ratio between the detected intensity of the reference antibody and the detected intensity of the target antibody, the detected amount of the reference antibody calculated from the mass spectrum can be multiplied by certain times, converted to a standard amount for the target antibody. The resulting value can be compared to the detected amount of the target antibody obtained from the mass spectra, thereby accomplishing determination of the concentration of the target antibody.

The program may be stored in a computer-readable storage medium. The storage medium storing the program according to the present embodiment may be the same as or different from a storage medium storing data for performing mass spectrometry, which is to be described below. The program stored in the storage medium may be read and run by a computer system. Herein, the "computer system" includes OS (Operating System) and peripheral apparatus hardware. The "computer-readable storage medium" includes transportable storage media such as flexible disk, magneto-optical disk, optical disk, and memory card, and storage devices such as internal hard disk of a computer system. The "computer-readable storage medium" may include a medium that holds a program temporarily and dynamically (like a communications line for transmitting a program via a network, such as internet, and via a telecommunications line, such as a telephone line) as well as a medium that holds a program for a certain length of time (like an internal volatile memory of a computer system such as a server and/or a client responsible for communications). The program may be used in combination with another program already stored in the computer system, to cause processing to be executed.

### [Storage Medium Storing Data for Performing Mass Spectrometry]

The present embodiment can also provide a computer-readable storage medium that stores data for performing mass spectrometry, for use in the method according to the present embodiment. The data may include, but not particularly limited to, data of parent ion, fragment ion, and expected retention time for one or more peptide fragments having an amino acid sequence characteristic of the antibody, and voltage for each member of triple quadrupole (first quadrupole, second quadrupole, third quadrupole). The data may also include information with regard to a particular antibody.

The data of the expected retention time, voltage, and the like is a numerical value variable depending on the device and the measurement conditions used, for example, and therefore, it is preferable that proper data for the device be provided. For a numerical value variable depending on the conditions, the range of variation is also preferably provided for better understanding of a person skilled in the art.

### [Method Package]

The present embodiment can also provide a method package that includes a storage medium storing the above program and/or data, for detection and quantification of a target antibody based on liquid chromatography-mass spectrometry (LC-MS).

Herein, the "method package" contains analysis conditions for liquid chromatography-mass spectrometry of a particular analyte and/or a program for target antibody quantification, in a readable form, and is distributable by itself. Importing the data contained in the method package to LC-MS allows for analysis to be performed under optimum measurement conditions determined by in-depth study. Running the program contained in the method package allows for accomplishing target antibody quantification. The method package can also contain instruction literature for the above-described storage medium.

Measurement with mass spectrometry enables highly accurate analysis, but it requires entirely different analysis conditions for each ion of interest. Setting proper analysis conditions is very important but also very difficult at the same time, requiring an enormous amount of time for setting the conditions. Preparing such conditions in advance allows for enhancing convenience for users who actually perform mass spectrometry.

The method package may contain data that is common for a plurality of mass spectrometers, or may contain various data suitable for analysis by a particular mass spectrometer.

The storage medium or the method package can be provided in combination with the above-described kit according to the present embodiment, or separately from the kit.

### [Examples]

Next, a more detailed description will be given of the present invention referring to Examples, which are not intended to limit the scope of the present invention.

### <Experiment 1>

Investigation was conducted to see if mass spectrometry could simultaneously identify a plurality of antibodies digested by nSMOL with protease. An antibody fractionated from serum was digested by nSMOL. The resulting peptide fragments were subjected to mass spectrometry for sequence identification. As the serum, a human standard reagent (a mixed sample from ten subjects, manufactured by Innovative Research, under the trade name of "Pooled Human AB Serum Plasma Derived") was used. As the mass spectrometer, LCMS-9030 (manufactured by Shimadzu) was used, and the MS spectra obtained were output in mzML file format and then analyzed with Peaks Studio version 10.5. Because individual human-derived antibody sequence information (antibody amino-acid sequence information) does not exist, spectrum matching and searching was conducted using public database (IMGT database).

Antibody digestion by nSMOL was carried out by using "nSMOL Antibody BA Kit" (manufactured by Shimadzu), in the below manner.
(1) Dilute 10 µL of serum with about 10 times the amount of PBS + 0.1% OTG.
(2) Add 25 µL of Immunoglobulin collection resin (support with Protein A immobilized inside pores, pore size 100 nm) to the diluted serum to obtain a liquid mixture.
(3) Stir the obtained liquid mixture gently with a vortex for 5 minutes.
(4) Collect the whole content of the liquid mixture on Ultra-free low-protein binding Durapore PVDF (0.22 µm) (centrifugal separation-type ultrafiltration filter).
(5) Perform centrifugation to remove the supernatant (10000 g, 1 minute).
(6) Add 300 µL of PBS + 0.1% OTG, followed by centrifugation to remove the supernatant (10000 g, 1 minute).
(7) Repeat Step (6).
(8) For surfactant (OTG) removal, add 300 µL of PBS, followed by centrifugation to remove the supernatant (10000 g, 1 minute).
(9) Repeat Step (8).
(10) Add 100 µL of Enhanced reaction solution.
(11) Add 5 µL of FG beads DART (diameter, 200 nm) having chemically-modified trypsin immobilized thereto (microparticles having a surface to which the protease is immobilized).
(12) Allow reaction to proceed for 5 hours under saturated vapor pressure at 50°C with gentle stirring.
(13) Add 10 µL of 10% formic acid to the reaction liquid to stop the reaction.
(14) Perform centrifugal filtration (10000 g, 1 minute) to recover a solution.
(15) Leave a tube containing the solution to stand on a magnetic stand for about 1 minute to remove excess magnetic beads from the solution.
(16) Perform LCMS analysis on the solution.

As a comparative example, peptide fragments resulting from antibody digestion with pepsin (manufactured by Promega, under the trade name of "Pepsin") or IdeS enzyme (manufactured by Promega, under the trade name of "IdeS Protease") were subjected to mass spectrometry. Results are shown in Table 1.

**[Table 1]**

| | Pepsin method | IdeS protease method | nSMOL |
|---|---|---|---|
| Serum amount | 10 mL | 10 mL | 10 µL |
| Number of identified peptides | 368 | 653 | 1257 |
| Number of identified IgG | 18 | 52 | 142 |
| Number of identified De novo sequences | 1554 | 2539 | 7660 |
| Number of identified hypermutable regions CDR3 | 0 | 5 | 31 |

It was verified that nSMOL allowed for simultaneous identification of a plurality of antibodies. nSMOL allowed for identification of more sequences from less serum than the pepsin method or the IdeS protease method.

### <Experiment 2>

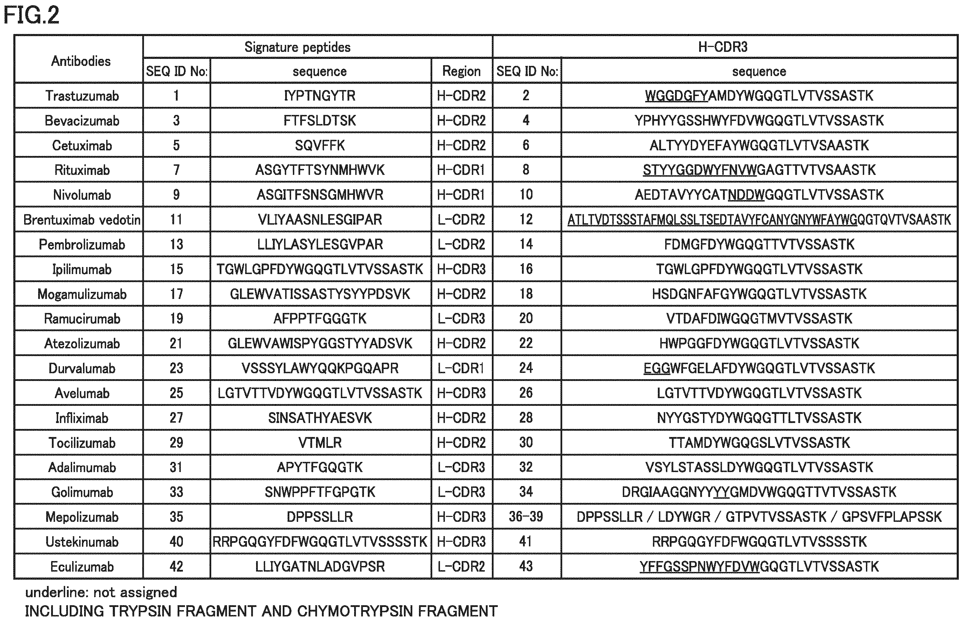

Further investigation was conducted to see if mass spectrometry could simultaneously identify a plurality of antibodies digested by nSMOL, using a monoclonal antibody having a known sequence. Twenty monoclonal antibodies including trastuzumab, bevacizumab, cetuximab, rituximab, nivolumab, brentuximab, pembrolizumab, ipilimumab, mogamulizumab, ramucirumab, atezolizumab, durvalumab, avelumab, infliximab, tocilizumab, adalimumab, golimumab, mepolizumab, ustekinumab, and eculizumab were mixed with human serum (manufactured by Innovative Research, under the trade name of "Pooled Human AB Serum Plasma Derived"). The samples thus obtained were digested by nSMOL as in Experiment 1, and the resulting peptide fragments were subjected to mass spectrometry. As the mass spectrometer, LCMS-9030 (manufactured by Shimadzu) was used. The MS spectra obtained were output in mzML file format, followed by use of Peaks Studio version 10.5, and spectrum matching and searching was conducted using in-house monoclonal antibody FASTA database. As a result, signature peptides and CDR3 sequences for all the antibodies used were identified. This suggested that mass spectrometry using nSMOL was universally applicable to antibody quantification. The signature peptide sequences and CDR3 sequences (SEQ ID NOs: 1 to 43) of the antibodies are shown in Fig. 2.

### <Experiment 3>

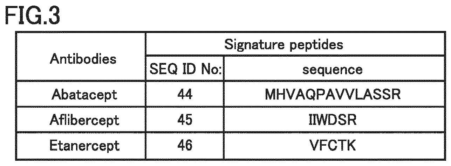

Investigation was conducted to determine variation between quantified values from individual analysis of antibodies and quantified values from simultaneous analysis of antibodies. A comparison was made between a value from quantification of a single sample to which a single antibody was added, and a value from quantification of a multiplex sample to which a plurality of antibodies were added. The single sample was a sample containing one of 21 monoclonal antibodies or Fc fusion proteins, namely brentuximab, cetuximab, rituximab, infliximab, atezolizumab, bevacizumab, pembrolizumab, trastuzumab, eculizumab, mepolizumab, tocilizumab, avelumab, durvalumab, ipilimumab, nivolumab, ramucirumab, adalimumab, golimumab, abatacept, aflibercept, and etanercept, mixed in human serum, and the multiplex sample was a sample containing all the above antibodies and Fc fusion proteins. Antibodies and Fc fusion proteins were each mixed in an amount of 50 µg/mL. The signature peptide sequences of the antibodies are shown in Fig. 2 and Fig. 3. In the following, the monoclonal antibody and the Fc fusion protein may be collectively called "antibody".

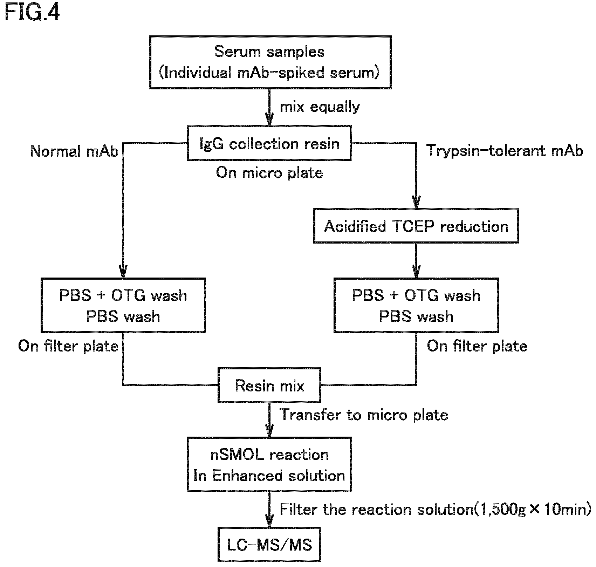

Each sample was subjected to digestion by improved nSMOL (Fig. 4) described below. This improved nSMOL was different from Experiment 2, in the following respects: for treating many samples, the reaction was performed in a microplate; for simplification of centrifugation process, a microplate equipped with built-in filter was used; for analyzing a protease-resistant antibody, antibodies were pretreated under strong acidic reducing conditions; and antibodies with strong acidic reduction treatment and antibodies without the treatment were analyzed together for an enhanced protease treatment efficiency.

Specific procedure is as follows:
(1) Dilute 10 µL of each sample with 90 µL of PBS + 0.1% OTG.
(2) Transfer the diluted sample to a low adsorption microplate (manufactured by Eppendorf, under the trade name of "Protein LoBind Plate"), followed by adding 25 µL of IgG collection resin (support with Protein A immobilized inside pores, pore size 100 nm).
(3) Divide the IgG collection resin into equal parts. Wash one of these directly with PBS + 0.1% OTG, and subject the other to acidic reduction treatment (250 mM TCEP-HCl) before washing. Washing was performed using a microplate equipped with built-in filter (manufactured by Whatman, "UNIFILTER filter plate", pore size of 20 µm), followed by disposing waste liquid with an aspiration manifold.
(4) Suspend each of the IgG collection resins contained in the microplate equipped with built-in filter, into Enhanced reaction solution, and put them in the same well of a low adsorption microplate, and thereby combining the resins from two conditions.
(5) Add 10 µL of FG beads Trypsin DART (diameter, 200 nm) (microparticles having a surface to which the protease is immobilized).
(6) Allow reaction to proceed for 5 hours under saturated vapor pressure at 50°C with gentle stirring.
(7) After reaction, add 10 µL of 10% formic acid to stop the reaction, and collect on a microplate equipped with built-in filter (manufactured by Nihon Pall Ltd., "AcroPrep advance 96-well Filter plate", pore size 200 nm).
(8) Perform centrifugation (1500 g, 10 minutes) to recover a solution containing peptide fragments.

LCMS was carried out under the conditions described below.
[LC] NexeraX2 system (manufactured by Shimadzu)
   Column: Shim-pack GISS C18 (50 mm x 2.1 mm)
   Column temperature: 50°C
   Solvent A: 0.1% formic acid/water
   Solvent B: 0.1% formic acid/acetonitrile
   Gradient: 1% B (1 minute)/1 to 50% B (5 minutes)/95% B (1.5 minutes)/1% B (10.5 minutes)
   Flow rate: 0.4 mL/minute
   Injection amount: 10 µL
[MS] LCMS-9030 (manufactured by Shimadzu)
   Ionization: ESI Positive
   DL temperature: 250°C
   Heat block temperature: 400°C
   Interface temperature: 300°C
   Nebulizer gas: 3 L/minute
   Drying gas: 10 L/minute
   Heating gas: 10 L/minute
   Mass spectrometer and analysis method were the same as in Experiment 2.

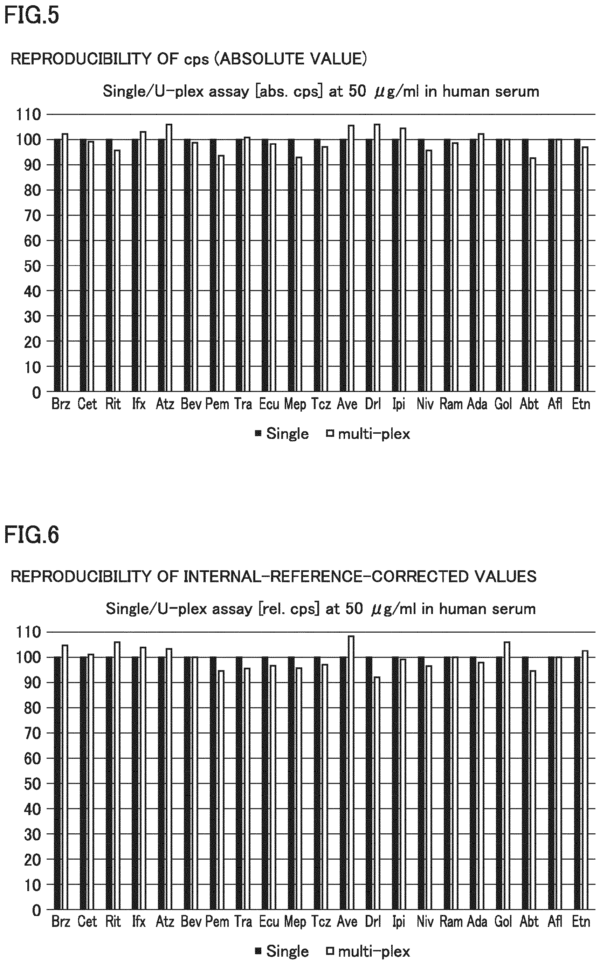

First, investigation was conducted to verify sufficiently high calibration curve reproducibility and accuracy for a single sample (N = 3) and a multiplex sample (N = 3). In the comparison between the single sample and the multiplex sample, ion counts (absolute number) and values after correction based on internal reference were used. Results of comparison between ion counts (cps) obtained from mass spectrometry of the single sample and cps of each antibody from mass spectrometry of the multiplex sample are shown in Fig. 5. Results of comparison between the detected amount for the single sample after correction based on an internal reference and the detected amount of each antibody in the multiplex sample after correction based on an internal reference are shown in Fig. 6. As the internal reference, P14R synthetic peptide (PPPPPPPPPPPPPPR: SEQ ID NO: 47) was used. Fig. 5 and Fig. 6 show relative values of each antibody in the multiplex sample, with respect to the value for the single sample defined as 100. The quantification accuracy for individual analysis of antibodies and for simultaneous analysis of antibodies was 6.7% (0.62% to 15.9%, the median was 6.5%). The variation range in accordance with validation guidance criteria from the United States Food and Drug Administration (FDA) is ±15%. A high accuracy was verified for the quantified values obtained by simultaneous mass spectrometry of a plurality of antibodies. It was shown that a plurality of antibodies, even clinical samples from a living body, for example, can be simultaneously quantified by mass spectrometry.

### <Experiment 4>

The ratio between a detected intensity of an antibody and a detected intensity of another antibody was determined. Using a multiplex sample containing all the 21 monoclonal antibodies and Fc fusion proteins described in Experiment 3 added to serum, the ratio of cps of an antibody to cps of trastuzumab was calculated. The ratio between a detected intensity of an antibody and a detected intensity of another antibody can also be calculated by using an antibody other than trastuzumab as a reference.
The antibody protease digestion method and mass spectrometry conditions are the same as in Experiment 3. For the multiplex sample, four concentrations were studied, each with five independent measurements (N = 5). This set of conditions satisfy FDA guidance.

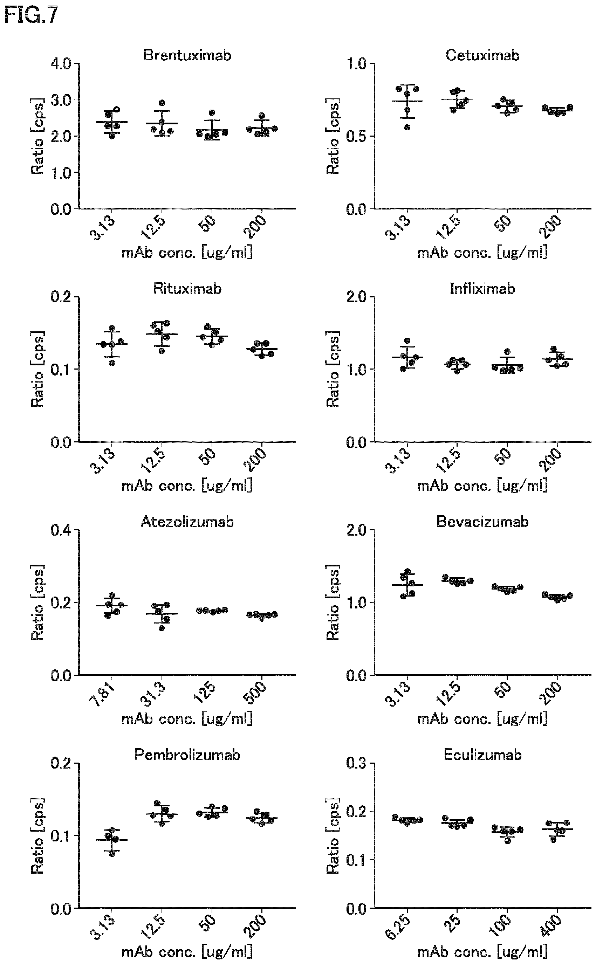

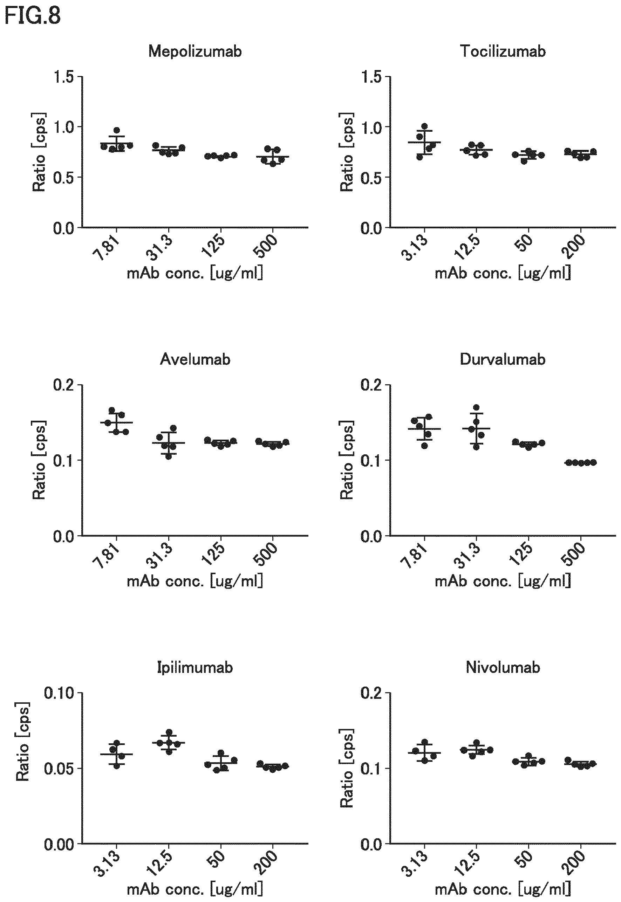

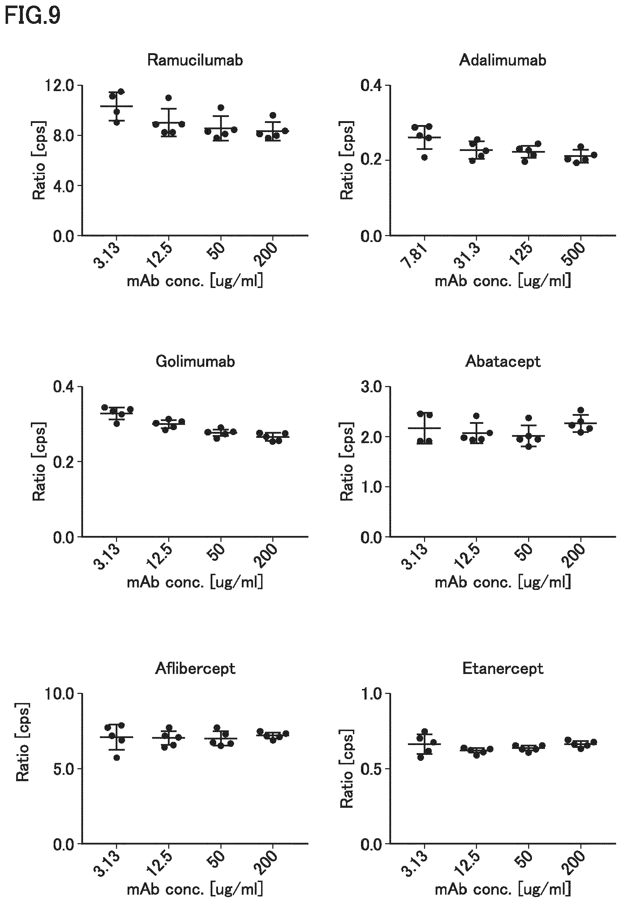

It was shown that the ratio of cps of an antibody to cps of trastuzumab (that is, the ratio between the detected intensity of a reference antibody and the detected intensity of a target antibody) was constant regardless of the antibody concentrations (Fig. 7 to Fig. 9). Use of this ratio (constant) can allow for quantification of an antibody other than trastuzumab using a calibration curve created with trastuzumab. The cps ratio of an antibody to another antibody may be the average value or the median value from a plurality of experiments.

### <Experiment 5>

An antibody (target antibody) contained in a clinical specimen was quantified based on a calibration curve created with trastuzumab (reference antibody). The clinical specimen was either serum from a human treated with ipilimumab administration (N = 71) or serum from a human treated with pembrolizumab administration (N = 95). Quantification was performed in the same manner as in Experiment 3. A trastuzumab calibration curve was created using a dilution series method (1000 µg/mL of trastuzumab was added to human serum, which was diluted to make 2-fold dilution series for creating a calibration curve). The trastuzumab calibration curve was corrected based on the ratio between trastuzumab detected intensity and ipilimumab detected intensity calculated in Experiment 4. Based on the corrected calibration curve, the ipilimumab concentration was determined. In the same manner, the trastuzumab calibration curve was corrected based on the ratio between trastuzumab detected intensity and pembrolizumab detected intensity to determine the pembrolizumab concentration.

To check the reproducibility of the quantified value, ipilimumab contained in the same clinical specimen was quantified based on the calibration curve created with ipilimumab. The ipilimumab calibration curve was created using a dilution series method (1000 µg/mL of ipilimumab was added to human serum, which was diluted to make 2-fold dilution series for creating a calibration curve). In the same manner, pembrolizumab contained in the clinical specimen was quantified based on the calibration curve created with pembrolizumab.

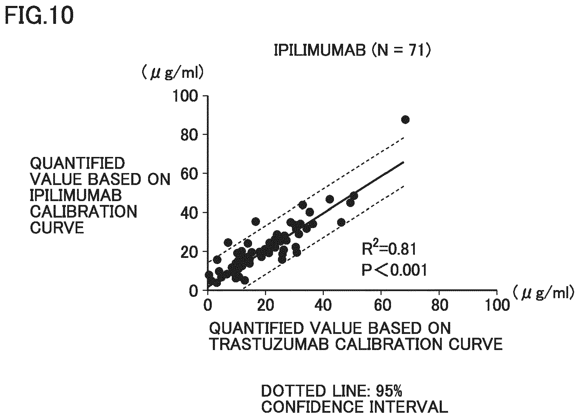

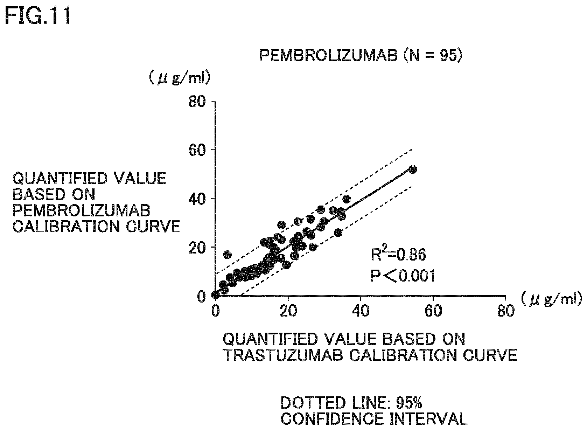

A comparison was made between the ipilimumab concentration quantified based on the trastuzumab calibration curve, and the ipilimumab concentration quantified based on the ipilimumab calibration curve. Pearson product-moment correlation analysis of the numerical values obtained from the two methods produced R² = 0.81, indicating a high reproducibility of both methods (Fig. 10). Similarly, a comparison was made between the pembrolizumab concentration quantified based on the trastuzumab calibration curve, and the pembrolizumab concentration quantified based on the pembrolizumab calibration curve. Pearson product-moment correlation analysis of the numerical values obtained from the two methods produced R² = 0.86, indicating a high reproducibility of both methods (Fig. 11). It was proven that use of the ratio between the detected intensity of the reference antibody and the detected intensity of the target antibody allows for determining concentrations of a plurality of target antibodies contained in a clinical specimen with respect to a single reference antibody.

### <Experiment 6>

It was verified based on the results of Experiment 5 whether antibodies other than trastuzumab and Fc fusion proteins could be used as the reference proteins (namely biological proteins). That is, an antibody (target antibody) contained in a clinical specimen was quantified based on a calibration curve created with any one of the reference proteins indicated below. The clinical specimen was either serum from a human treated with ipilimumab administration (N = 71) or serum from a human treated with pembrolizumab administration (N = 74). Quantification was performed in the same manner as in Experiment 3. A reference protein calibration curve was created using a dilution series method (1000 µg/mL of reference protein was added to the collected human serum, which was diluted to make 2-fold dilution series for creating a calibration curve sample). The reference protein calibration curve was corrected based on the ratio between the reference protein detected intensity and the ipilimumab detected intensity calculated in the same manner as in Experiment 4. Based on the corrected calibration curve, the ipilimumab concentration was determined. In the same manner, the calibration curve created with the reference protein was corrected based on the ratio between the reference protein detected intensity and the pembrolizumab detected intensity to determine the pembrolizumab concentration.

### (Used Reference Proteins)

Trastuzumab (Tra), brentuximab vedotin (Bre), cetuximab (Cet), rituximab (Rit), infliximab (Ifx), atezolizumab (Atz), bevacizumab (Bev), pembrolizumab (Pem), eculizumab (Ecu), mepolizumab (Mep), tocilizumab (Toc), avelumab (Ave), durvalumab (Dyr), ipilimumab (Ipi), nivolumab (Niv), ramucirumab (Ram), adalimumab (Ada), golimumab (Gol), abatacept (Abt), and etanercept (Etn) were used.

To check the reproducibility of the quantified value, ipilimumab contained in the same clinical specimen was quantified based on the calibration curve created with ipilimumab. The ipilimumab calibration curve was created using a dilution series method (1000 µg/mL of ipilimumab was added to human serum, which was diluted to make 2-fold dilution series for creating a calibration curve). In the same manner, pembrolizumab contained in the clinical specimen was quantified based on the calibration curve created with pembrolizumab.

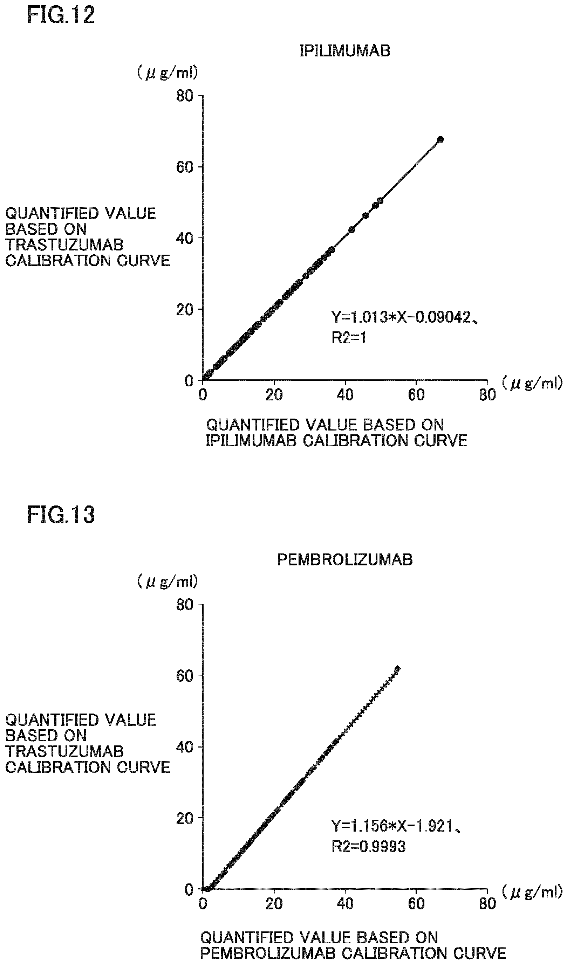

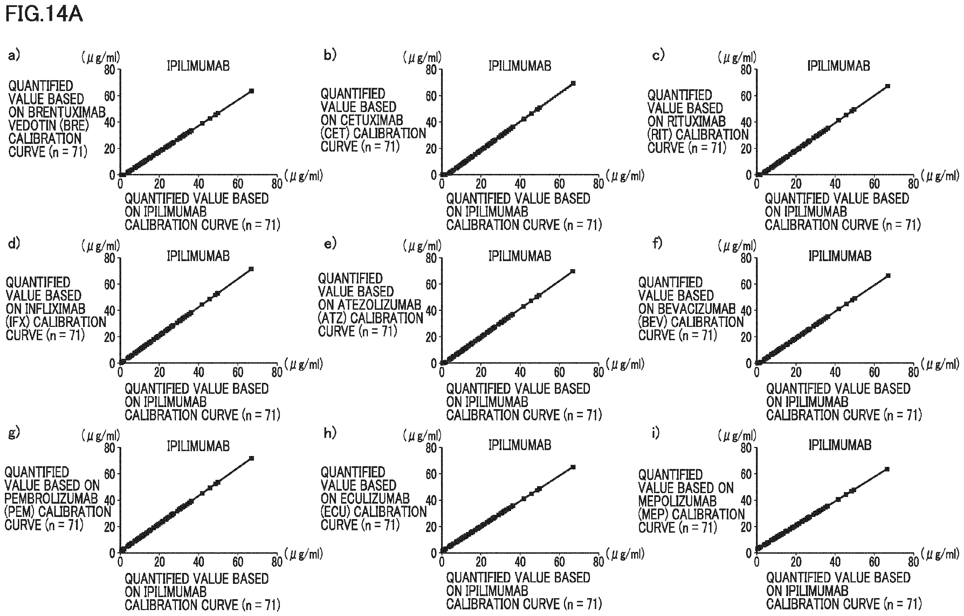

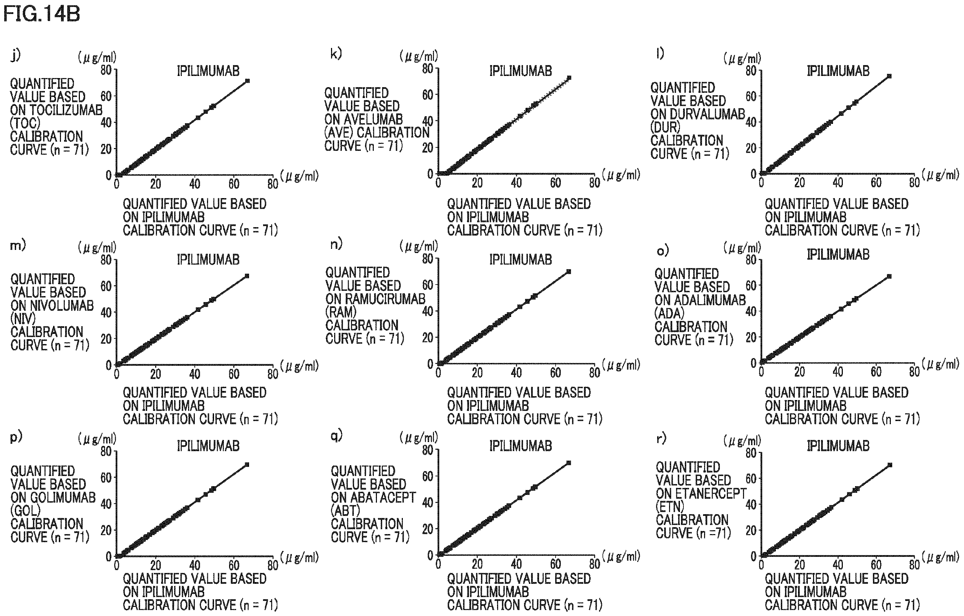

A comparison was made between the ipilimumab concentration quantified based on the reference protein calibration curve, and the ipilimumab concentration quantified based on the ipilimumab calibration curve (Figs. 12, 14A, and 14B). Pearson product-moment correlation analysis of the numerical values obtained from the two methods produced an R² value of 0.99 or more in all the reference proteins, indicating a high reproducibility (Table 2).

**[Table 2]**

| Reference mAb | Goodness of fit r² |
|---|---|
| Brenzuximab vedotin | 0.998 |
| Cetuximab | 0.9993 |
| Rituximab | 0.9997 |
| Infliximab | 1 |
| Atezolizumab | 0.9999 |
| Sevacizumab | 0.9999 |
| Pembrolizumab | 1 |
| Trastuzumab | 1 |
| Eculizumab | 1 |
| Mepolizumab | 1 |
| Tocilizumab | 0.9995 |
| Avelumab | 0.9974 |
| Durvalumab | 0.9999 |
| Nivolumab | 1 |
| Ramucirumab | 0.9999 |
| Adalimumab | 1 |
| Golimumab | 0.999 |
| Abatacept | 1 |
| Etanercept | 1 |

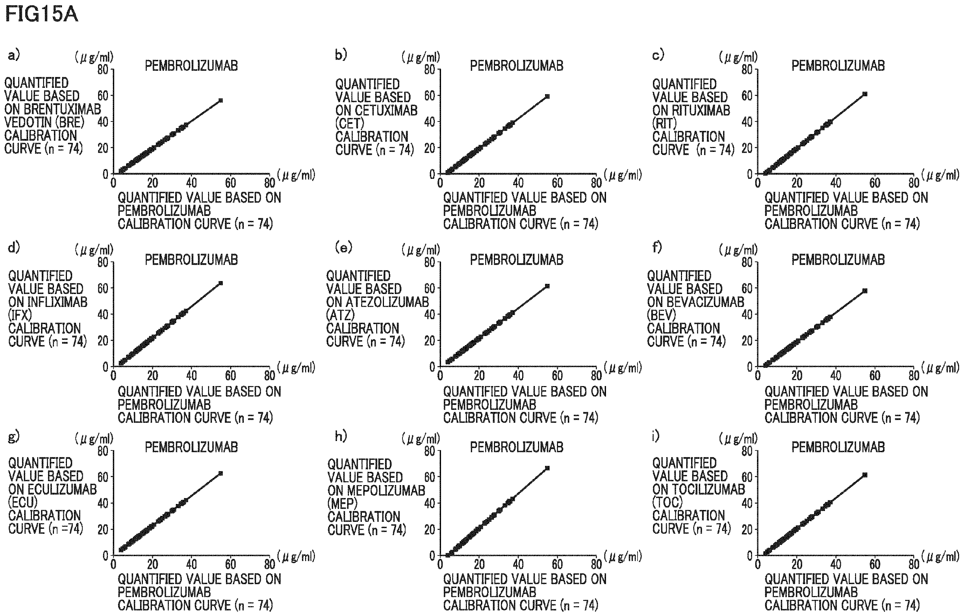

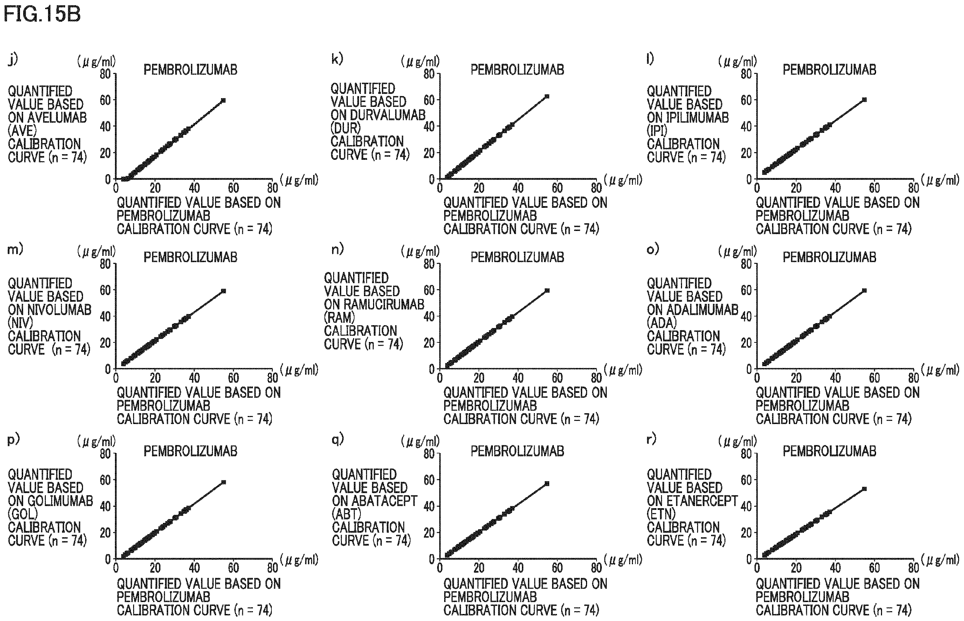

Similarly, a comparison was made between the pembrolizumab concentration quantified based on the reference protein calibration curve, and the pembrolizumab concentration quantified based on the pembrolizumab calibration curve (Figs. 13, 15A, and 15B). Pearson product-moment correlation analysis of the numerical values obtained from the two methods produced an R² value of 0.99 or more in all the reference proteins, indicating a high reproducibility (Table 3). It was proven from the above results that use of the ratio between the detected intensity of the reference protein and the detected intensity of the target antibody allows for determining concentrations of a plurality of target antibodies contained in a clinical specimen with respect to a single reference antibody.

**[Table 3]**

| Reference mAb | Goodness of fit r² |
|---|---|
| Brenzuximab vedotin | 1 |
| Cetuximab | 1 |
| Rituximab | 1 |
| Infliximab | 1 |
| Atezolizumab | 1 |
| Sevacizumab | 1 |
| Trastuzumab | 1 |
| Eculizumab | 1 |
| Mepolizumab | 0.9999 |
| Tocilizumab | 1 |
| Avelumab | 0.9991 |
| Durvalumab | 1 |
| Ipilimumab | 1 |
| Nivolumab | 1 |
| Ramucirumab | 1 |
| Adalimumab | 1 |
| Golimumab | 1 |
| Abatacept | 1 |
| Etanercept | 1 |

### [Aspects]

As will be appreciated by those skilled in the art, the example embodiments and Examples described above are specific examples of the below aspects.

### (First Item)

An antibody analysis method according to one aspect includes:
digesting a biological protein and a target antibody with a protease;
performing mass spectrometry of peptide fragments of the biological protein and the target antibody obtained by the digesting; and
quantifying the target antibody based on a ratio between a detected intensity of the biological protein and a detected intensity of the target antibody obtained by the mass spectrometry,
the digesting with a protease including:
   immobilizing the biological protein and the target antibody inside pores of a support; and
   bringing a microparticle close to the support, the microparticle having a diameter larger than a diameter of the pores and having a surface to which a protease is immobilized,
wherein the biological protein has a domain capable of being immobilized inside the pores.

The antibody analysis method according to the first item enables antibody quantification with mass spectrometry without creating a calibration curve for an antibody of interest.

### (Second Item)

The antibody analysis method according to the first item further includes:
performing mass spectrometry of the biological protein at a predetermined concentration and the target antibody at a predetermined concentration; and
determining in advance the ratio between a detected intensity of the biological protein and a detected intensity of the target antibody, based on results of the mass spectrometry.

Determining in advance the ratio between a detected intensity of the biological protein (such as reference antibody, for example) and a detected intensity of the target antibody allows for easy and simple quantification of the target antibody.

### (Third Item)

In the antibody analysis method according to the first item or the second item, the biological protein includes a reference antibody or an Fc fusion protein.

The reference antibody and the Fc fusion protein are of general applicability and readily available, and are therefore suitable for use as the biological protein.

### (Fourth Item)

In the antibody analysis method according to the third item, the reference antibody is a monoclonal antibody.

A monoclonal antibody is readily available and easy to determine the concentration and the amino acid sequence, and is therefore suitable for use as the reference antibody.

### (Fifth Item)

In the antibody analysis method according to the third item, the reference antibody is selected from the group consisting of trastuzumab, bevacizumab, cetuximab, rituximab, nivolumab, brentuximab, pembrolizumab, ipilimumab, mogamulizumab, ramucirumab, atezolizumab, durvalumab, avelumab, infliximab, tocilizumab, adalimumab, golimumab, mepolizumab, ustekinumab, and eculizumab.

The above antibodies are of general applicability and readily available, and are therefore suitable for use as the reference antibody.

### (Sixth Item)

In the antibody analysis method according to the third item, the Fc fusion protein is selected from the group consisting of abatacept, aflibercept, and etanercept.

The Fc fusion protein is of general applicability and readily available, and is therefore suitable for use as the biological protein.

### (Seventh Item)

In the antibody analysis method according to any one of the first item to the sixth item, the target antibody is an antibody contained in a biological sample.

The antibody analysis method according to the seventh item enables quantification of a target antibody contained in a biological sample obtained from a subj ect.

### (Eighth Item)

In the antibody analysis method according to any one of the first item to the seventh item, the target antibody includes at least two antibodies.

The antibody analysis method according to the eighth item enables quantification of a plurality of target antibodies with respect to a single biological protein (such as reference antibody, for example). The two or more antibodies may be quantified simultaneously.

### (Ninth Item)

In the antibody analysis method according to any one of the first item to the eighth item, the target antibody includes an antibody produced in a living body.

The antibody analysis method according to the ninth item enables quantification of an antibody produced in a living body and difficult to create a calibration curve for.

### (Tenth Item)

An antibody analysis method according to one aspect includes:
digesting a biological protein and a sample containing at least two target antibodies, with a protease;
performing mass spectrometry of peptide fragments of the biological protein and the target antibodies obtained by the digesting; and
quantifying each of the target antibodies based on a ratio between a detected intensity of the biological protein and a detected intensity of the each of the target antibodies obtained by the mass spectrometry,
wherein the biological protein is a monoclonal antibody.

The antibody analysis method according to the tenth item enables antibody quantification with mass spectrometry without creating a calibration curve using an antibody of interest.

### (Eleventh Item)

A kit according to one aspect is a kit for use in the antibody analysis method according to any one of the first item to the tenth item, the kit including:
the biological protein;
the microparticle having a surface to which a protease is immobilized; and
the support provided with a pore.

The kit according to the eleventh item enables easy and simple implementation of the method according to the first item.

### (Twelfth Item)

A program according to one aspect is a program for use in the antibody analysis method according to any one of the first item to the tenth item, the program causing a processor to execute processes of:
receiving a mass spectrum output from a mass spectrometer;
retrieving a ratio between a detected intensity of the biological protein and a detected intensity of the target antibody, from a storage member,
correcting a detected amount of the biological protein calculated from the mass spectrum; and
calculating an amount of the target antibody.

The program according to the twelfth item enables easy and simple processing of target antibody quantification based on a ratio between a detected intensity of the biological protein and a detected intensity of the target antibody.

### REFERENCE SIGNS LIST

10 microparticle, 11 spacer, 15 protease, 20 support, 21 linker, 25 antibody, 29 pore

## Claims

1. An antibody analysis method, comprising:
digesting a biological protein and a target antibody with a protease;
performing mass spectrometry of peptide fragments of the biological protein and the target antibody obtained by the digesting; and
quantifying the target antibody based on a ratio between a detected intensity of the biological protein and a detected intensity of the target antibody obtained by the mass spectrometry,
the digesting with a protease including:
immobilizing the biological protein and the target antibody inside pores of a support; and
bringing a microparticle close to the support, the microparticle having a diameter larger than a diameter of the pores and having a surface to which a protease is immobilized,
wherein the biological protein has a domain capable of being immobilized inside the pores.

2. The antibody analysis method according to claim 1, further comprising:
performing mass spectrometry of the biological protein at a predetermined concentration and the target antibody at a predetermined concentration; and
determining in advance the ratio between a detected intensity of the biological protein and a detected intensity of the target antibody, based on results of the mass spectrometry.

3. The antibody analysis method according to claim 1 or 2, wherein the biological protein includes a reference antibody or an Fc fusion protein.

4. The antibody analysis method according to claim 3, wherein the reference antibody is a monoclonal antibody.

5. The antibody analysis method according to claim 3, wherein the reference antibody is selected from the group consisting of trastuzumab, bevacizumab, cetuximab, rituximab, nivolumab, brentuximab, pembrolizumab, ipilimumab, mogamulizumab, ramucirumab, atezolizumab, durvalumab, avelumab, infliximab, tocilizumab, adalimumab, golimumab, mepolizumab, ustekinumab, and eculizumab.

6. The antibody analysis method according to claim 3, wherein the Fc fusion protein is selected from the group consisting of abatacept, aflibercept, and etanercept.

7. The antibody analysis method according to claim 1 or 2, wherein the target antibody is an antibody contained in a biological sample.

8. The antibody analysis method according to claim 1 or 2, wherein the target antibody includes at least two antibodies.

9. The antibody analysis method according to claim 1 or 2, wherein the target antibody includes an antibody produced in a living body.

10. An antibody analysis method, comprising:
digesting a biological protein and a sample containing at least two target antibodies, with a protease;
performing mass spectrometry of peptide fragments of the biological protein and the target antibodies obtained by the digesting; and
quantifying each of the target antibodies based on a ratio between a detected intensity of the biological protein and a detected intensity of the each of the target antibodies obtained by the mass spectrometry,
wherein the biological protein is a monoclonal antibody.

11. A kit for use in the antibody analysis method according to claim 1 or 2, the kit comprising:
the biological protein;
the microparticle having a surface to which a protease is immobilized; and
the support provided with a pore.

12. A program for use in the antibody analysis method according to claim 1 or 2, the program causing a processor to execute processes of:
receiving a mass spectrum output from a mass spectrometer;
retrieving a ratio between a detected intensity of the biological protein and a detected intensity of the target antibody, from a storage member;
correcting a detected amount of the biological protein calculated from the mass spectrum; and
calculating an amount of the target antibody.
